# EUROPEAN PATENT APPLICATION

(11) **EP 4 825 482 A1**
(43) Date of publication of application: **21.10.2026**
(21) Application number: 24903314.3
(22) Date of filing: 11.10.2024
(51) Int. Cl.: B01F 35/221, B01F 21/00, B01F 21/10, B01F 23/53, B01F 35/75, B01F 35/80, B01F 35/222, C12M 1/00, C12N 1/00, B01F 101/44

(54) **SOLUTION PREPARATION DEVICE, METHOD FOR OPERATING SOLUTION PREPARATION DEVICE, AND PROGRAM FOR OPERATING SOLUTION PREPARATION DEVICE**

(30) Priority: 12.12.2023 JP 2023209539
(71) Applicant: FUJIFILM Corporation, Tokyo 106-8620 (JP)
(72) Inventor: YAMAMOTO, Takashi, Tokyo 106-8620 (JP); HASEGAWA, Masataka, Tokyo 106-8620 (JP); INABA, Tatsuya, Tokyo 106-8620 (JP)
(74) Representative: Klunker IP Patentanwälte PartG mbB
(86) International application number: PCT/JP2024/036538
(87) International publication number: WO 2025/126651

(57) **Abstract**

A solution preparation device including a first pump that causes a solvent to continuously flow into a mixing container, a powder feeder that continuously supplies a powder to the mixing container, a stirrer that stirs the solvent and the powder in the mixing container, and a second pump that causes a solution having a set concentration, prepared by mixing the solvent and the powder, to continuously flow out from the mixing container, the solution preparation device including: a processor that controls driving of the first pump, the powder feeder, the stirrer, and the second pump, the processor being configured to periodically change at least any one of an inflow amount of the solvent into the mixing container by the first pump, an outflow amount of the solution from the mixing container by the second pump, or a stirring rotation speed by the stirrer.

## Description

### Technical Field

The technology of the present disclosure relates to a solution preparation device, an operation method of a solution preparation device, and an operation program of a solution preparation device.

### Related Art

For example, in a bioprocess such as culturing Chinese hamster ovary cells into which an antibody gene has been incorporated to obtain an antibody drug, various solutions are required. WO2021/123248A discloses a solution preparation device that continuously supplies a solvent and a powder to a mixing container and continuously prepares a solution having a set concentration by stirring and mixing the solvent and the powder in the mixing container. The mixing container is connected to a bioprocess container, and the solution is continuously supplied from the mixing container to the bioprocess container. The solvent is, for example, purified water such as pure water, the powder is, for example, a powdered culture medium, and the solution is, for example, a culture medium (culture solution). In addition, the bioprocess container is, for example, a culture tank.

### SUMMARY OF THE INVENTION

### Technical Problem

A small amount of undissolved powder is present in the mixing container. In a case where the amount of the undissolved powder is unnecessarily increased by being deposited on a liquid surface of the solution and/or a bottom surface of the mixing container, a continuous powder dissolution step does not reach a steady state, and a concentration of the solution is unstable. However, in WO2021/123248A, no measures are taken to solve the problem of the instability of the solution concentration due to the unnecessary deposition of the undissolved powder.

One embodiment according to the technology of the present disclosure provides a solution preparation device, an operation method of a solution preparation device, and an operation program of a solution preparation device, which can contribute to stabilization of a solution concentration by undissolved powder.

### Solution to Problem

A solution preparation device according to the present disclosure is a solution preparation device including a first pump that causes a solvent to continuously flow into a mixing container, a powder feeder that continuously supplies a powder to the mixing container, a stirrer that stirs the solvent and the powder in the mixing container, and a second pump that causes a solution having a set concentration, which is prepared by mixing the solvent and the powder, to continuously flow out from the mixing container, the solution preparation device comprising: a processor that controls driving of the first pump, the powder feeder, the stirrer, and the second pump, in which the processor is configured to periodically change at least any one of an inflow amount of the solvent into the mixing container by the first pump, an outflow amount of the solution from the mixing container by the second pump, or a stirring rotation speed by the stirrer.

It is preferable that a fluctuation amplitude and a fluctuation period of at least any one of the inflow amount, the outflow amount, or the stirring rotation speed are set to values at which a concentration fluctuation of the solution caused by the periodic fluctuation of at least any one of the inflow amount, the outflow amount, or the stirring rotation speed is within an allowable range.

It is preferable that a concentration fluctuation index CFI represented by the following equation is equal to or less than an amount L of a liquid in the mixing container. Equation: $CFI = \left\{\left(V\times T\right)/60\right\} \times A$

Here, V is a set amount of the inflow amount or the outflow amount, A is a fluctuation amplitude of at least any one of the inflow amount or the outflow amount, and T is a fluctuation period of at least any one of the inflow amount or the outflow amount.

It is preferable that the processor is configured to further periodically change a stirring rotation direction by the stirrer in a case where the processor is configured to periodically change the stirring rotation speed.

It is preferable that the solvent is purified water, the powder is a powdered culture medium, and the solution is a culture medium.

An operation method of a solution preparation device of the present disclosure is an operation method of a solution preparation device including a first pump that causes a solvent to continuously flow into a mixing container, a powder feeder that continuously supplies a powder to the mixing container, a stirrer that stirs the solvent and the powder in the mixing container, and a second pump that causes a solution having a set concentration, which is prepared by mixing the solvent and the powder, to continuously flow out from the mixing container, the method comprising: controlling driving of the first pump, the powder feeder, the stirrer, and the second pump; and periodically changing at least any one of an inflow amount of the solvent into the mixing container by the first pump, an outflow amount of the solution from the mixing container by the second pump, or a stirring rotation speed by the stirrer.

An operation program of a solution preparation device of the present disclosure is an operation program of a solution preparation device including a first pump that causes a solvent to continuously flow into a mixing container, a powder feeder that continuously supplies a powder to the mixing container, a stirrer that stirs the solvent and the powder in the mixing container, and a second pump that causes a solution having a set concentration, which is prepared by mixing the solvent and the powder, to continuously flow out from the mixing container, the program causing a computer to execute a process comprising: controlling driving of the first pump, the powder feeder, the stirrer, and the second pump; and periodically changing at least any one of an inflow amount of the solvent into the mixing container by the first pump, an outflow amount of the solution from the mixing container by the second pump, or a stirring rotation speed by the stirrer.

### Advantageous Effects of Invention

According to the technology of the present disclosure, it is possible to provide a solution preparation device, an operation method of a solution preparation device, and an operation program of a solution preparation device, which can contribute to stabilization of a solution concentration by undissolved powder.

### BRIEF DESCRIPTION OF THE DRAWINGS

FIG. 1 is a diagram showing a culture medium preparation device and a culture tank.
FIG. 2 is a block diagram showing a configuration of a control unit.
FIG. 3 is a block diagram showing a processing unit of a CPU.
FIG. 4 is a graph showing a temporal change in an inflow amount of pure water into a mixing container.
FIG. 5 is a graph showing a temporal change in a concentration of a culture medium.
FIG. 6 is a graph showing a method of setting a fluctuation amplitude and a fluctuation period of the inflow amount of the pure water into the mixing container.
FIG. 7 is a diagram showing a concentration fluctuation index, a concentration fluctuation index in which a concentration fluctuation of the culture medium in the mixing container falls within an allowable range, and a condition of the amount of liquid in the mixing container.
FIG. 8 is a block diagram showing a processing unit of a CPU of a second embodiment.
FIG. 9 is a graph showing a temporal change in an outflow amount of the culture medium from the mixing container.
FIG. 10 is a block diagram showing a processing unit of a CPU of a third embodiment.
FIG. 11 is a graph showing a temporal change in a stirring rotation speed.
FIG. 12 is a graph showing an aspect in which a stirring rotation direction is also periodically changed in addition to the periodic fluctuation of the stirring rotation speed.
FIG. 13 is a table showing Examples and Comparative Examples.

### DESCRIPTION OF EMBODIMENTS

### [First embodiment]

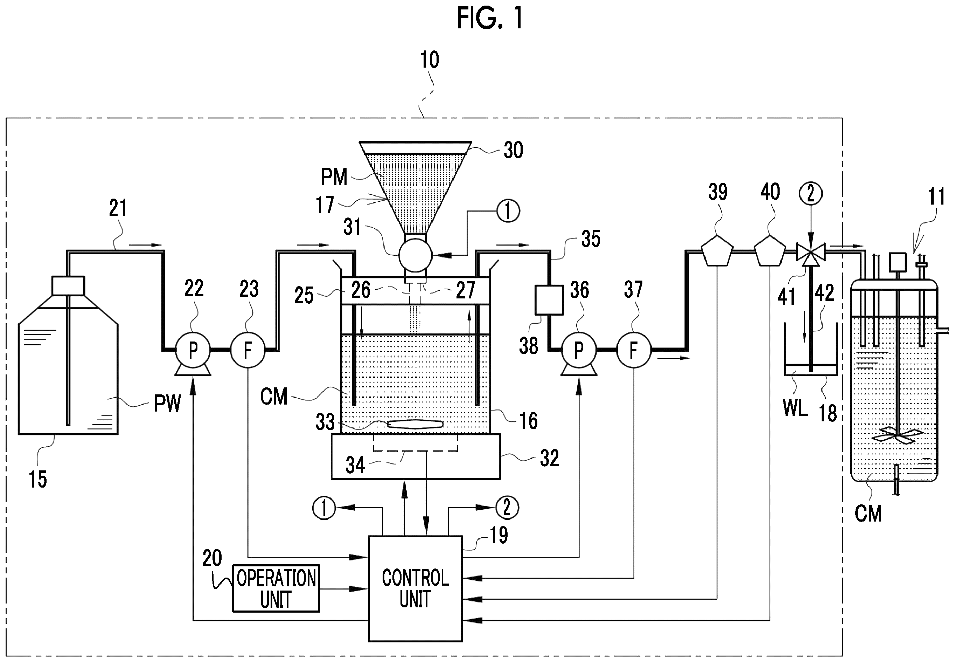

As shown in FIG. 1 as an example, a culture medium preparation device 10 prepares a culture medium CM. The culture medium preparation device 10 is connected to the culture tank 11, and continuously supplies the prepared culture medium CM to the culture tank 11. The culture medium preparation device 10 is an example of a "solution preparation device" according to the technology of the present disclosure. In addition, the culture medium CM is an example of a "solution" according to the technology of the present disclosure. It is noted that in FIG. 1, an example in which the culture medium preparation device 10 is directly connected to the culture tank 11 has been described, but the present disclosure is not limited thereto. A tank for temporarily storing the culture medium CM may be provided between the culture medium preparation device 10 and the culture tank 11.

For example, Chinese hamster ovary cells into which an antibody gene has been incorporated are seeded in the culture tank 11. In the culture tank 11, the Chinese hamster ovary cells are cultured in the culture medium CM for a determined culture period, for example, one month. As described above, since the culture medium CM is continuously supplied to the culture tank 11 from the culture medium preparation device 10, the culture of the Chinese hamster ovary cells performed in the culture tank 11 is a perfusion culture. The culture tank 11 is connected to a purification device (not shown). The purification device purifies an antibody produced by Chinese hamster ovary cells in the culture process, and yields the antibody as an active pharmaceutical ingredient of an antibody drug.

The culture medium preparation device 10 comprises a pure water storage tank 15, a mixing container 16, a powdered culture medium supply machine 17, a waste liquid recovery tank 18, a control unit 19, an operation unit 20, and the like.

The pure water storage tank 15 stores pure water PW. The pure water PW is an example of a "solvent" and "purified water" according to the technology of the present disclosure. One end of a pure water supply line 21 is connected to the pure water storage tank 15. The other end of the pure water supply line 21 is connected to the mixing container 16. A pure water supply pump 22 and a pure water flow meter 23 are provided in the pure water supply line 21. The pure water supply pump 22 is driven and controlled by the control unit 19. The pure water supply pump 22 is an example of a "first pump" according to the technology of the present disclosure. In a case where the pure water supply pump 22 is driven, the pure water PW in the pure water storage tank 15 is supplied to the mixing container 16 through the pure water supply line 21. The pure water flow meter 23 is disposed downstream of the pure water supply pump 22, and measures an inflow amount of the pure water PW that passes through the pure water supply line 21 and flows into the mixing container 16. The pure water flow meter 23 outputs the measured inflow amount to the control unit 19. The control unit 19 controls the driving of the pure water supply pump 22 such that the inflow amount of the pure water flow meter 23 is a set inflow amount. The set inflow amount is an example of a "set amount of an inflow amount or an outflow amount" according to the technology of the present disclosure.

The mixing container 16 is a container for mixing the pure water PW and the powdered culture medium PM to generate the culture medium CM having a set concentration, and has, for example, a wide cylindrical shape. The upper portion of the mixing container 16 is sealed with a lid 25. A through-hole 26 is formed in a center portion of the lid 25. A discharge port 27 for the powdered culture medium PM of the powdered culture medium supply machine 17 is aseptically connected to an upper end part of the through-hole 26. As a result, the mixing container 16 and the powdered culture medium supply machine 17 are aseptically connected. The powdered culture medium PM discharged from the discharge port 27 is supplied to the mixing container 16 through the through-hole 26. The powdered culture medium PM is an example of a "powder" according to the technology of the present disclosure.

The powdered culture medium supply machine 17 is an example of a "powder feeder" according to the technology of the present disclosure. The powdered culture medium supply machine 17 is composed of a funnel-shaped hopper 30 that stores the powdered culture medium PM and a feeder 31 that continuously discharges the powdered culture medium PM in the hopper 30 from the discharge port 27. The hopper 30 has a funnel shape, and a sterile bag (not shown) in which the powdered culture medium PM is accommodated is set in an upper opening of the hopper 30. The powdered culture medium PM is introduced into the hopper 30 from the sterile bag without coming into contact with outside air. As a result, the sterile state of the powdered culture medium PM is maintained.

The feeder 31 is attached to a lower portion of the hopper 30 in which a diameter is narrowed. The feeder 31 is driven and controlled by the control unit 19, and continuously discharges the set amount of the powdered culture medium PM from the discharge port 27. The feeder 31 is, for example, a screw feeder (also referred to as an auger feeder) that continuously discharges the powdered culture medium PM in a set amount from the discharge port 27 by rotationally driving a spiral screw with a motor under the control of the control unit 19. Although not shown, a stirrer for crushing rat holes generated by long-term use is introduced into the hopper 30. The inner wall of the hopper 30 may be subjected to a sliding treatment for improving the discharge properties of the powdered culture medium PM. In addition, the feeder 31 is not limited to the screw feeder, and may be a table feeder (also referred to as a circle feeder), a rotary feeder, a belt feeder, a vibrating feeder, or the like.

The mixing container 16 is installed on the stirrer 32. A stirring bar 33 is put into the mixing container 16. The stirring bar 33 has an elongated cocoon-like shape in which both end portions are rounded and a central portion is slightly swollen. A magnet is built in the stirring bar 33. The stirrer 32 is driven and controlled by the control unit 19. The stirrer 32 generates a magnetic force for rotating the stirring bar 33. The stirrer 32 rotates the stirring bar 33 at a set stirring rotation speed to mix the pure water PW and the powdered culture medium PM to obtain a culture medium CM. It should be noted that, without being limited to the stirring bar 33, the pure water PW and the powdered culture medium PM may be mixed by rotating a stirring blade (also referred to as a stirring vane).

A weighing scale 34 is built in the stirrer 32. The weighing scale 34 measures the weight of the mixing container 16 installed on the stirrer 32. The weighing scale 34 outputs the measured weight to the control unit 19. The control unit 19 controls the driving of the powdered culture medium supply machine 17 such that the weight is a set amount. It should be noted that the supply amount of the powdered culture medium PM related to the weight of the mixing container 16 is set based on the set concentration of the culture medium CM to be prepared, the outflow amount of the culture medium CM to the culture tank 11, and the like.

One end of a culture medium supply line 35 is connected to the mixing container 16. The other end of the culture medium supply line 35 is connected to the culture tank 11. A culture medium supply pump 36 and a culture medium flow meter 37 are provided in the culture medium supply line 35. The culture medium supply pump 36 is driven and controlled by the control unit 19. The culture medium supply pump 36 is an example of a "second pump" according to the technology of the present disclosure. In a case where the culture medium supply pump 36 is driven, the culture medium CM in the mixing container 16 is supplied to the culture tank 11 through the culture medium supply line 35. The culture medium flow meter 37 is disposed downstream of the culture medium supply pump 36, and measures the outflow amount of the culture medium CM that passes through the culture medium supply line 35 and flows out to the culture tank 11. The culture medium flow meter 37 outputs the measured outflow amount to the control unit 19. The control unit 19 controls the driving of the culture medium supply pump 36 such that the outflow amount of the culture medium flow meter 37 is the set outflow amount. The set outflow amount is an example of a "set amount of inflow amount or outflow amount" according to the technology of the present disclosure.

The inflow amount of the pure water PW into the mixing container 16 and the outflow amount of the culture medium CM from the mixing container 16 are set to values such that the amount of the liquid in the mixing container 16 does not change from the beginning to the end. More specifically, the outflow amount of the culture medium CM is set according to the amount obtained by combining the inflow amount of the pure water PW and the supply amount of the powdered culture medium PM. In practice, the weight of the mixing container 16 is measured by the weighing scale 34, and the inflow amount of the pure water PW and the outflow amount of the culture medium CM are set such that the measured weight is the set amount.

A filter 38 is disposed upstream of the culture medium supply pump 36 of the culture medium supply line 35. The filter 38 removes unnecessary substances in the culture medium CM. The unnecessary substance is, for example, an undissolved powdered culture medium PM, and the like. In a case where the amount of the unnecessary substances such as the undissolved powdered culture medium PM can be set to be less than the allowable amount, the filter 38 may not be provided.

A conductivity meter 39 and a hydrogen-ion exponent meter 40 are disposed downstream of the culture medium flow meter 37 in the culture medium supply line 35. In addition, a waste liquid line 42 is connected to the downstream of the hydrogen-ion exponent meter 40 of the culture medium supply line 35 via a three-way valve 41. The three-way valve 41 is driven and controlled by the control unit 19. The waste liquid recovery tank 18 is connected to the waste liquid line 42. The waste liquid recovery tank 18 collects the waste liquid WL such as the culture medium CM that is not at the set concentration or the culture medium CM having a hydrogen-ion exponent of an abnormal value.

The conductivity meter 39 measures the electrical conductivity (unit: mS/cm) of the culture medium CM passing through the culture medium supply line 35 as a substitute for the concentration of the culture medium CM. The conductivity meter 39 outputs the measured conductivity to the control unit 19. The control unit 19 determines whether or not the culture medium CM has the set concentration based on the conductivity from the conductivity meter 39. In a case where it is determined that the culture medium CM is not at the set concentration, the control unit 19 sets the flow path of the three-way valve 41 to the waste liquid line 42 side, and discharges the waste liquid WL, which is the culture medium CM not having the set concentration, to the waste liquid recovery tank 18. On the other hand, in a case where it is determined that the culture medium CM has the set concentration, the control unit 19 sets the flow path of the three-way valve 41 to the culture tank 11 side, and introduces the culture medium CM having the set concentration into the culture tank 11. In this way, only the culture medium CM having the set concentration is supplied to the culture tank 11. It is possible to prevent a problem such as the supply of the culture medium CM not having the set concentration to the culture tank 11 and the failure of the culture of the Chinese hamster ovary cells.

The hydrogen-ion exponent meter 40 measures the hydrogen-ion exponent of the culture medium CM that passes through the culture medium supply line 35. The hydrogen-ion exponent meter 40 outputs the measured hydrogen-ion exponent to the control unit 19. In a case where the hydrogen-ion exponent from the hydrogen-ion exponent meter 40 is an abnormal value, the control unit 19 sets the flow path of the three-way valve 41 to the waste liquid line 42 side, and discharges the waste liquid WL, which is the culture medium CM having an abnormal value of the hydrogen-ion exponent, to the waste liquid recovery tank 18.

The operation unit 20 is, for example, a touch panel, and receives various operation instructions from an operator of the culture medium preparation device 10. The various operation instructions include a preparation start instruction for the culture medium CM, a preparation end instruction for the culture medium CM, a forced stop instruction for the supply of the culture medium CM from the mixing container 16 to the culture tank 11, and the like.

The pure water storage tank 15, the mixing container 16, the hopper 30 and the feeder 31 of the powdered culture medium supply machine 17, and the waste liquid recovery tank 18 are disposable in one time, that is, so-called single-use. In addition, the pure water supply line 21, the culture medium supply line 35, the filter 38, the three-way valve 41, and the waste liquid line 42 are also single-use.

All flow paths from the pure water storage tank 15 to the culture tank 11 through the pure water supply line 21, the mixing container 16, and the culture medium supply line 35 are aseptically connected. In addition, the culture medium supply line 35, the three-way valve 41, and the waste liquid line 42 are also aseptically connected.

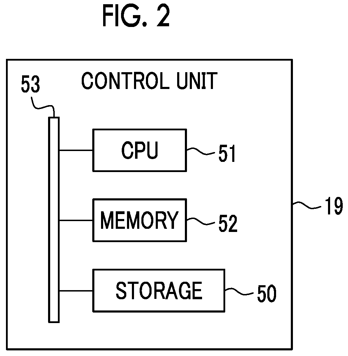

As shown in FIG. 2 as an example, the control unit 19 comprises a storage 50, a central processing unit (CPU) 51, and a memory 52. The storage 50, the CPU 51, and the memory 52 are connected to each other through a busline 53. The control unit 19 is an example of a "computer" according to the technology of the present disclosure.

The storage 50 is a hard disk drive or a solid-state drive. The storage 50 stores, for example, a control program, such as an operating system, various application programs, and various types of data associated with these programs.

The memory 52 is a work memory for executing processing via the CPU 51. The CPU 51 loads a program stored in the storage 50 into the memory 52 and executes processing corresponding to the program. Therefore, the CPU 51 controls each unit of the computer comprehensively. The CPU 51 is an example of a "processor" according to the technology of the present disclosure. The memory 52 may be incorporated in the CPU 51.

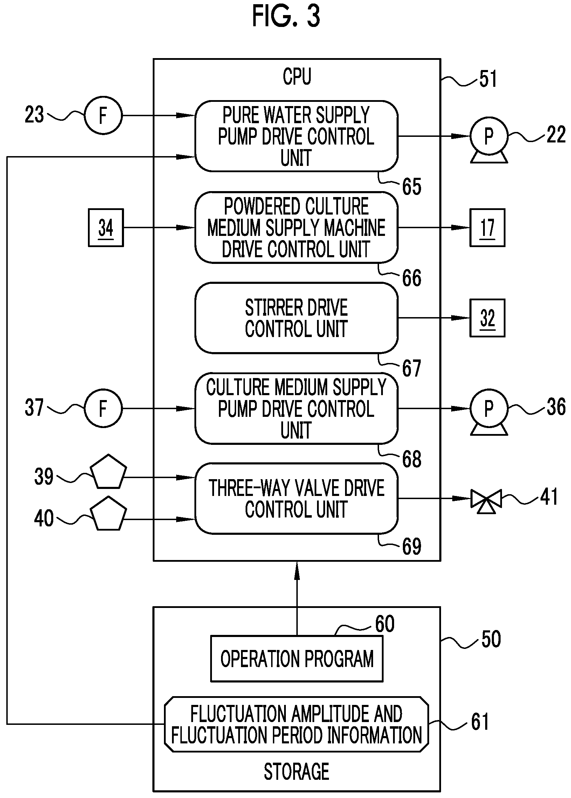

As shown in FIG. 3 as an example, the storage 50 stores an operation program 60. The operation program 60 is an example of an "operation program of a solution preparation device" according to the technology of the present disclosure. In addition to the operation program 60, fluctuation amplitude and fluctuation period information 61 is also stored in the storage 50.

In a case where the operation program 60 is activated, the CPU 51 functions as a pure water supply pump drive control unit 65, a powdered culture medium supply machine drive control unit 66, a stirrer drive control unit 67, a culture medium supply pump drive control unit 68, and a three-way valve drive control unit 69.

The pure water supply pump drive control unit 65 controls the drive of the pure water supply pump 22 based on the inflow amount of the pure water PW into the mixing container 16 measured by the pure water flow meter 23. In addition, the pure water supply pump drive control unit 65 controls the drive of the pure water supply pump 22 based on the fluctuation amplitude and fluctuation period information 61. The drive control of the pure water supply pump 22 based on the fluctuation amplitude and fluctuation period information 61 will be described later.

The powdered culture medium supply machine drive control unit 66 controls the drive of the powdered culture medium supply machine 17 based on the weight of the mixing container 16 measured by the weighing scale 34. The stirrer drive control unit 67 controls the drive of the stirrer 32 such that the stirring rotation speed of the stirring bar 33 is the set rotation speed.

The culture medium supply pump drive control unit 68 controls the drive of the culture medium supply pump 36 based on the outflow amount of the culture medium CM from the mixing container 16 measured by the culture medium flow meter 37. The three-way valve drive control unit 69 controls the drive of the three-way valve 41 based on the conductivity of the culture medium CM measured by the conductivity meter 39 and the hydrogen-ion exponent of the culture medium CM measured by the hydrogen-ion exponent meter 40.

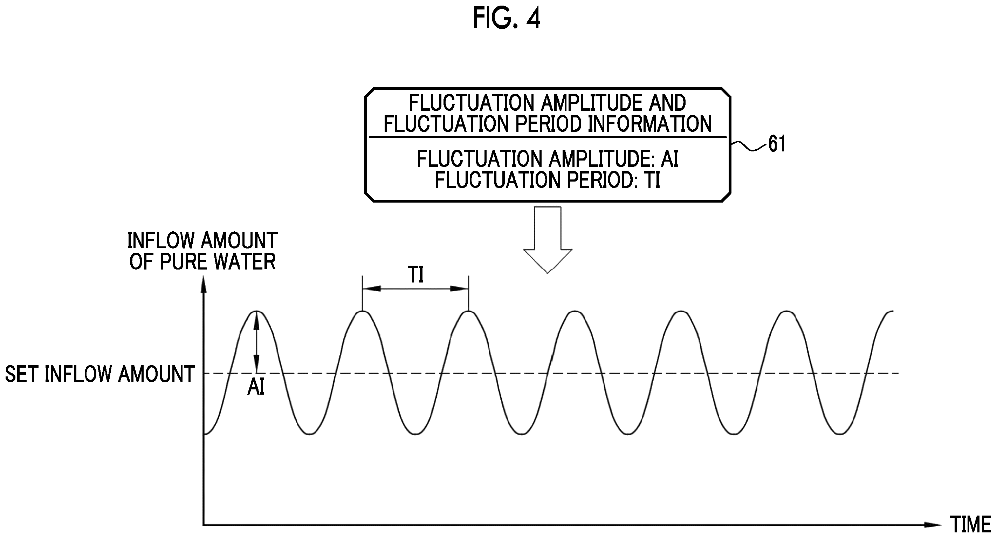

As shown in FIG. 4 as an example, the fluctuation amplitude AI and the fluctuation period TI of the inflow amount of the pure water PW into the mixing container 16 by the pure water supply pump 22 are registered in the fluctuation amplitude and fluctuation period information 61. The pure water supply pump drive control unit 65 periodically changes the inflow amount of the pure water PW into the mixing container 16 with the set inflow amount as a center, by using the fluctuation amplitude AI and the fluctuation period TI registered in the fluctuation amplitude and fluctuation period information 61. By periodically changing the inflow amount of the pure water PW into the mixing container 16 in this way, the liquid level of the liquid in the mixing container 16 also periodically moves up and down as a result. In addition, since the inflow amount of the pure water PW into the mixing container 16 is periodically fluctuated with the set inflow amount as a center, the inflow amount of the pure water PW into the mixing container 16 is the set inflow amount on average.

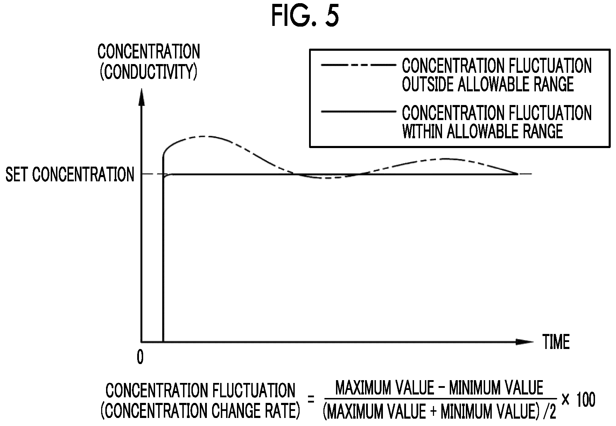

In a case where the inflow amount of the pure water PW into the mixing container 16 is periodically fluctuated, an appropriate stimulus is applied to the liquid in the mixing container 16. Therefore, the undissolved powdered culture medium PM is less likely to be deposited on the liquid surface of the culture medium CM in the mixing container 16 and/or the bottom surface of the mixing container 16, as compared with a case where no stimulus is applied to the liquid in the mixing container 16. In order to make it more difficult for the undissolved powdered culture medium PM to be deposited, the fluctuation amplitude AI may be increased or the fluctuation period TI may be lengthened. However, in a case where the fluctuation amplitude AI is increased or the fluctuation period TI is lengthened, a concentration fluctuation outside the allowable range occurs in the culture medium CM in the mixing container 16, as shown by a two-dot chain line in a graph of FIG. 5 as an example. On the contrary, in a case where the fluctuation amplitude AI is reduced and the fluctuation period TI is shortened, the concentration fluctuation of the culture medium CM in the mixing container 16 falls within the allowable range, as shown by a solid line in the graph of FIG. 5 as an example, but the effect of making it difficult for the undissolved powdered culture medium PM to be deposited is weakened. That is, there is an appropriate value for the fluctuation amplitude AI and the fluctuation period TI of the inflow amount of the pure water PW into the mixing container 16.

Here, the concentration fluctuation is, in other words, a rate of change in concentration, and is calculated by, for example, the following Equation (1). 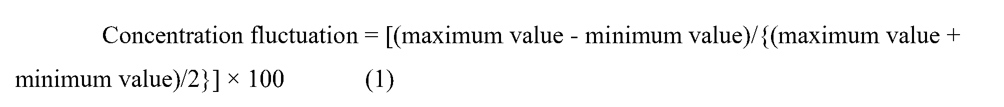 Concentration fluctuation = [(maximum value - minimum value)/{(maximum value + minimum value)/2}] × 100 (1)

The maximum value and the minimum value are a maximum value and a minimum value of the conductivity of the culture medium CM measured by the conductivity meter 39 until the concentration of the culture medium CM in the mixing container 16 reaches a steady state. The steady state is, for example, a state in which the concentration of the culture medium CM in the mixing container 16 is the set concentration ± α is continued for a set time.

In a case where the concentration fluctuation is large, it takes a long time for the concentration of the culture medium CM in the mixing container 16 to converge to the steady state, and the amount of the culture medium CM that is not the set concentration and is discharged as the waste liquid WL increases accordingly. The allowable range of the concentration fluctuation is set based on a limit amount of the amount of the culture medium CM that is not the set concentration and is discharged as the waste liquid WL. The allowable range of the concentration fluctuation is, for example, 3% or less.

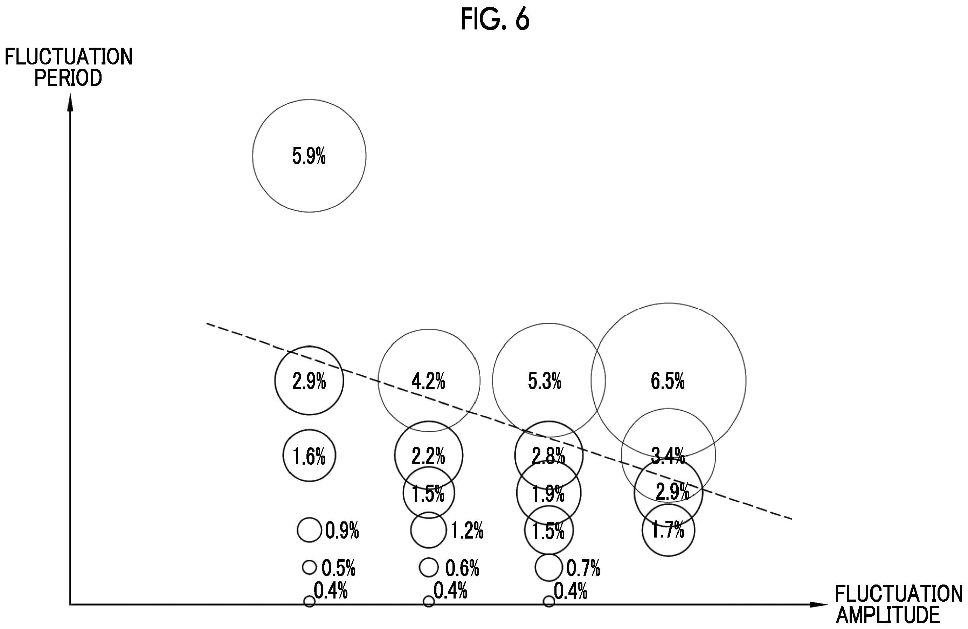

The operator performs a setting experiment described later before the culture medium preparation device 10 is operated, to set the fluctuation amplitude AI and the fluctuation period TI of the inflow amount of the pure water PW into the mixing container 16. That is, as shown in FIG. 6 as an example, the fluctuation amplitude AI and the fluctuation period TI are set to various values, the culture medium CM having the set concentration is actually prepared in the mixing container 16, and each concentration fluctuation is calculated. Then, the fluctuation amplitude AI and the fluctuation period TI are set to any value below the broken line in which the effect of making it difficult to deposit the undissolved powdered culture medium PM is sufficiently expected and the concentration fluctuation is within the allowable range. The operator stores the set fluctuation amplitude AI and the set fluctuation period TI in the storage 50 as fluctuation amplitude and fluctuation period information 61. In FIG. 6, the size of the circle represents the magnitude of the concentration fluctuation, and the numerical value in or beside the circle represents the concentration fluctuation.

In addition, in the setting experiment, the operator sets the amount of the liquid in the mixing container 16. Specifically, the amount of the liquid is set to gradually increase from a small value to a large value, and the culture medium CM is actually prepared in the mixing container 16 while periodically changing the inflow amount of the pure water PW into the mixing container 16 with the fluctuation amplitude AI and the fluctuation period TI set in advance. Then, the minimum value at which the effect of making it difficult to deposit the undissolved powdered culture medium PM is sufficiently expected is set as the amount of the liquid in the mixing container 16.

In order to make it difficult to deposit the undissolved powdered culture medium PM, it is preferable that the amount of the liquid in the mixing container 16 is large. However, in a case where the amount of the liquid in the mixing container 16 increases, the capacity of the mixing container 16 increases, and the installation area of the mixing container 16 increases. Therefore, the minimum value at which the effect of making it difficult to deposit the undissolved powdered culture medium PM is sufficiently expected is set as the amount of the liquid in the mixing container 16.

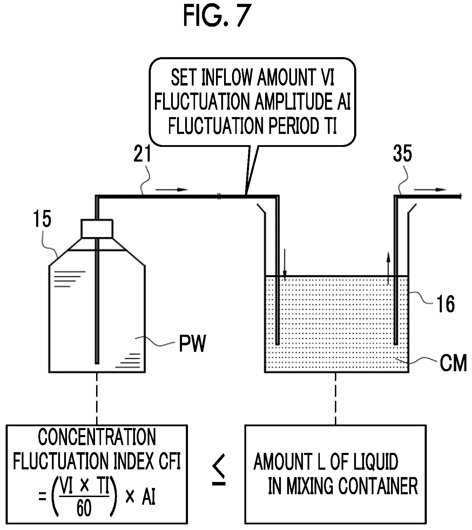

Furthermore, as shown in FIG. 7 as an example, the operator sets the set inflow amount VI of the pure water PW and the fluctuation amplitude AI and the fluctuation period TI of the inflow amount of the pure water PW into the mixing container 16 such that the concentration fluctuation index CFI is equal to or less than the amount L of the liquid in the mixing container 16 (CFI ≤ L). In a case where the concentration fluctuation index CFI is equal to or less than the amount L of the liquid in the mixing container 16, the concentration fluctuation of the culture medium CM in the mixing container 16 caused by the periodic fluctuation of the inflow amount of the pure water PW into the mixing container 16 falls within the allowable range. The concentration fluctuation index CFI is represented by the following equation (2A). $CFI = \left\{\left(VI\times TI\right)/60\right\} \times AI$ (2A)

Next, the actions obtained from the above-described configuration will be described. First, before the culture medium preparation device 10 is operated, the operator performs the setting experiment shown in FIG. 6, and the fluctuation amplitude AI and the fluctuation period TI of the inflow amount of the pure water PW into the mixing container 16 are set. The set fluctuation amplitude AI and the set fluctuation period TI are stored in the storage 50 as fluctuation amplitude and fluctuation period information 61 by the operator. In addition, the amount of the liquid in the mixing container 16 is also set by the operator. The set inflow amount VI of the pure water PW and the fluctuation amplitude AI and the fluctuation period TI of the inflow amount of the pure water PW into the mixing container 16 are set to values at which the concentration fluctuation index CFI is equal to or less than the amount L of the liquid in the mixing container 16.

The operator gives a preparation start instruction for the culture medium CM via the operation unit 20. As a result, the pure water supply pump 22 is driven under the control of the pure water supply pump drive control unit 65 to supply the pure water PW to the mixing container 16. At the same time, the powdered culture medium supply machine 17 is driven under the control of the powdered culture medium supply machine drive control unit 66 to supply the powdered culture medium PM to the mixing container 16. That is, the continuous supply of the pure water PW and the powdered culture medium PM to the mixing container 16 is started.

As shown in FIG. 4, the pure water supply pump drive control unit 65 causes the inflow amount of the pure water PW into the mixing container 16 to periodically fluctuate with the fluctuation amplitude AI and the fluctuation period TI of the fluctuation amplitude and fluctuation period information 61. In addition, the stirrer 32 is driven to rotate the stirring bar 33 at the set stirring rotation speed under the control of the stirrer drive control unit 67.

After a while after the start of the supply of the pure water PW and the powdered culture medium PM to the mixing container 16, the culture medium supply pump 36 is driven under the control of the culture medium supply pump drive control unit 68. The inflow amount of the pure water PW into the mixing container 16, the outflow amount of the culture medium CM from the mixing container 16, and the like are adjusted according to the weight measured by the weighing scale 34 such that the amount of the liquid in the mixing container 16 is a value set in the setting experiment.

The three-way valve drive control unit 69 determines whether or not the culture medium CM has the set concentration based on the conductivity measured by the conductivity meter 39. In a case where it is determined that the culture medium CM does not have the set concentration, the flow path of the three-way valve 41 is set to the waste liquid line 42 side under the control of the three-way valve drive control unit 69, and the waste liquid WL, which is the culture medium CM that does not have the set concentration, is discharged to the waste liquid recovery tank 18. On the other hand, in a case where it is determined that the culture medium CM has the set concentration, the flow path of the three-way valve 41 is set to the culture tank 11 side under the control of the three-way valve drive control unit 69, and the culture medium CM having the set concentration is introduced into the culture tank 11.

The continuous supply of the pure water PW and the powdered culture medium PM to the mixing container 16 and the continuous supply of the culture medium CM to the culture tank 11 are continued until the culture in the culture tank 11 is ended. In a case where the culture in the culture tank 11 is ended, the driving of the pure water supply pump 22, the powdered culture medium supply machine 17, the culture medium supply pump 36, and the like is stopped, and the supply of the pure water PW and the powdered culture medium PM to the mixing container 16 and the supply of the culture medium CM to the culture tank 11 are stopped.

As described above, the culture medium preparation device 10 comprises the pure water supply pump 22 that causes the pure water PW to continuously flow into the mixing container 16, the powdered culture medium supply machine 17 that continuously supplies the powdered culture medium PM to the mixing container 16, the stirrer 32 that stirs the pure water PW and the powdered culture medium PM in the mixing container 16, and the culture medium supply pump 36 that causes the culture medium CM having the set concentration, which is prepared by mixing the pure water PW and the powdered culture medium PM, to continuously flow out from the mixing container 16. The culture medium preparation device 10 comprises the CPU 51 that controls the driving of the pure water supply pump 22, the powdered culture medium supply machine 17, the stirrer 32, and the culture medium supply pump 36. The pure water supply pump drive control unit 65 of the CPU 51 periodically changes the inflow amount of the pure water PW into the mixing container 16 by the pure water supply pump 22. As a result, the undissolved powdered culture medium PM is less likely to be deposited on the liquid surface of the culture medium CM in the mixing container 16 and/or the bottom surface of the mixing container 16, and it is possible to contribute to the stabilization of the concentration of the culture medium CM.

As shown in FIGS. 5 and 6, the fluctuation amplitude AI and the fluctuation period TI of the inflow amount of the pure water PW into the mixing container 16 are set to values at which the concentration fluctuation of the culture medium CM caused by the periodic fluctuation of the inflow amount is within an allowable range. Therefore, it is possible to reduce the amount of the culture medium CM, which is not the set concentration, discharged as the waste liquid WL.

As shown in FIG. 7, the set inflow amount VI of the pure water PW and the fluctuation amplitude AI and the fluctuation period TI of the inflow amount of the pure water PW into the mixing container 16 are set such that the concentration fluctuation index CFI is equal to or less than the amount L of the liquid in the mixing container 16. Therefore, it is possible to keep the concentration fluctuation of the culture medium CM in the mixing container 16 caused by the periodic fluctuation of the inflow amount of the pure water PW into the mixing container 16 within an allowable range. As a result, it is possible to reduce the amount of the culture medium CM, which is not the set concentration, discharged as the waste liquid WL.

As shown in FIG. 1, the solvent is the pure water PW, the powder is the powdered culture medium PM, and the solution is the culture medium CM. Therefore, it is possible to prepare the culture medium CM having the set concentration, which is indispensable for the culture, in a short time.

### [Second embodiment]

In the first embodiment, an example in which the inflow amount of the pure water PW into the mixing container 16 is periodically fluctuated has been described, but the present disclosure is not limited thereto.

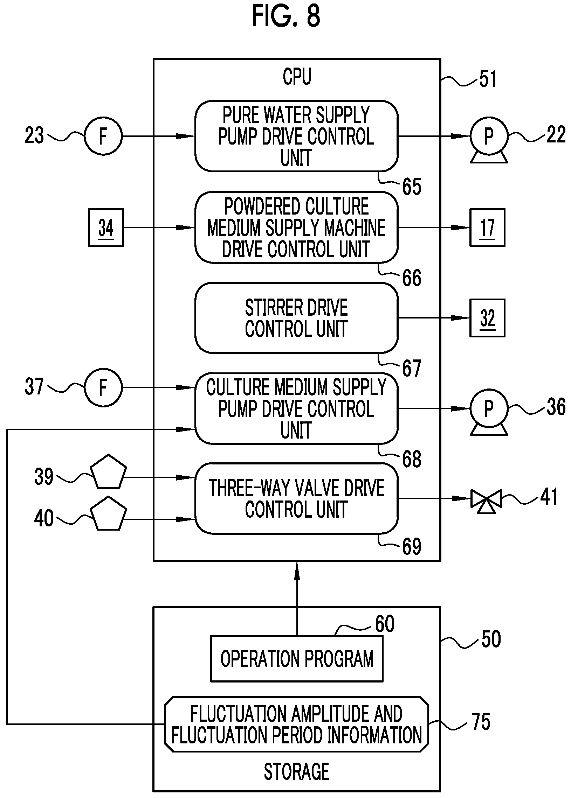

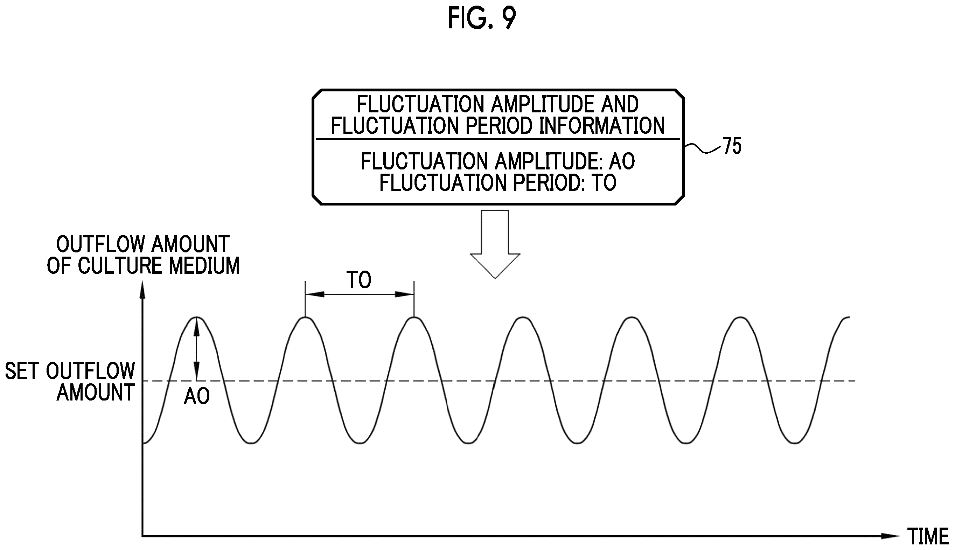

As shown in FIG. 8 as an example, in the second embodiment, fluctuation amplitude and fluctuation period information 75 is provided to the culture medium supply pump drive control unit 68. As shown in FIG. 9 as an example, fluctuation amplitude and fluctuation period information 75 is registered with a fluctuation amplitude AO and a fluctuation period TO of the outflow amount of the culture medium CM from the mixing container 16 by the culture medium supply pump 36. As in the first embodiment, the fluctuation amplitude AO and the fluctuation period TO are set to appropriate values in which the effect of making it difficult to deposit the undissolved powdered culture medium PM, which is clarified in the setting experiment, is sufficiently expected and the concentration fluctuation is within the allowable range. The culture medium supply pump drive control unit 68 periodically changes the inflow amount of the culture medium CM from the mixing container 16 with the set outflow amount as a center by using the fluctuation amplitude AO and the fluctuation period TO registered in the fluctuation amplitude and fluctuation period information 75. As in the case of the first embodiment, the inflow amount of the culture medium CM from the mixing container 16 is periodically fluctuated in this way, so that an appropriate stimulus is applied to the liquid in the mixing container 16. In addition, since the outflow amount of the culture medium CM from the mixing container 16 is periodically fluctuated with the set outflow amount as a center, the outflow amount of the culture medium CM from the mixing container 16 is the set outflow amount on average.

As described above, in the second embodiment, the culture medium supply pump drive control unit 68 of the CPU 51 periodically changes the outflow amount of the culture medium CM from the mixing container 16 by the culture medium supply pump 36. Even with this method, the undissolved powdered culture medium PM is less likely to be deposited on the liquid surface of the culture medium CM in the mixing container 16 and/or the bottom surface of the mixing container 16, and it is possible to contribute to the stabilization of the concentration of the culture medium CM.

The concentration fluctuation index CFI in the second embodiment is represented by the following Equation (2B). $CFI = \left\{\left(VO\times TO\right)/60\right\} \times AO$ (2B)

Here, VO is the set outflow amount of the pure water PW.

The first embodiment and the second embodiment described above may be implemented in combination. That is, the inflow amount of the pure water PW into the mixing container 16 may be periodically fluctuated, and the outflow amount of the culture medium CM from the mixing container 16 may be periodically fluctuated. In this case, the fluctuation period TI of the inflow amount of the pure water PW into the mixing container 16 and the fluctuation period TO of the outflow amount of the culture medium CM from the mixing container 16 are set to be the same. In addition, the waveform of the inflow amount of the pure water PW into the mixing container 16 and the waveform of the outflow amount of the culture medium CM from the mixing container 16 are set to be in phase with each other to reinforce each other.

### [Third embodiment]

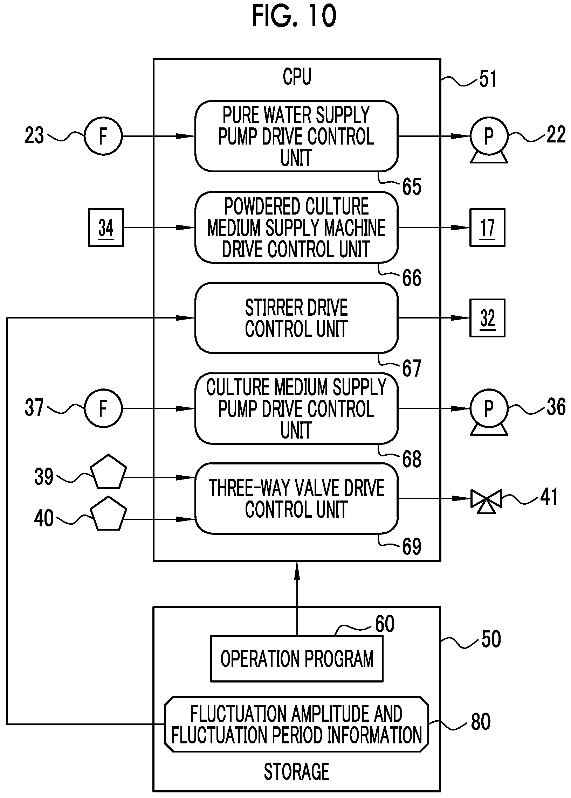

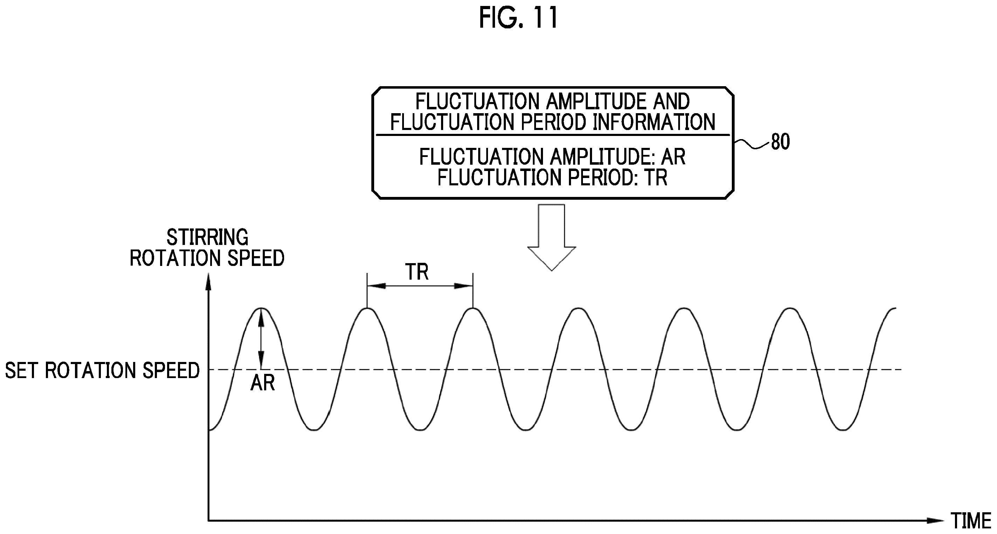

As shown in FIG. 10 as an example, in the third embodiment, fluctuation amplitude and fluctuation period information 80 is provided to the stirrer drive control unit 67. As shown in FIG. 11 as an example, in the fluctuation amplitude and fluctuation period information 80, a fluctuation amplitude AR and a fluctuation period TR of the stirring rotation speed of the stirring bar 33 by the stirrer 32 are registered. As in the first and second embodiments, the fluctuation amplitude AR and the fluctuation period TR are set to appropriate values that can sufficiently expect the effect of making it difficult to deposit the undissolved powdered culture medium PM, which is clarified in the setting experiment, and that the concentration fluctuation is within the allowable range. The stirrer drive control unit 67 periodically changes the stirring rotation speed of the stirring bar 33 by the stirrer 32 around the set rotation speed with the fluctuation amplitude AR and the fluctuation period TR registered in the fluctuation amplitude and fluctuation period information 80. As in the first and second embodiments, by periodically changing the stirring rotation speed in this way, an appropriate stimulus is applied to the liquid in the mixing container 16. In addition, since the stirring rotation speed is periodically fluctuated around the set rotation speed, the average stirring rotation speed is the set rotation speed.

As described above, in the third embodiment, the stirrer drive control unit 67 of the CPU 51 periodically changes the stirring rotation speed of the stirring bar 33 by the stirrer 32. Even with this method, the undissolved powdered culture medium PM is less likely to be deposited on the liquid surface of the culture medium CM in the mixing container 16 and/or the bottom surface of the mixing container 16, and it is possible to contribute to the stabilization of the concentration of the culture medium CM.

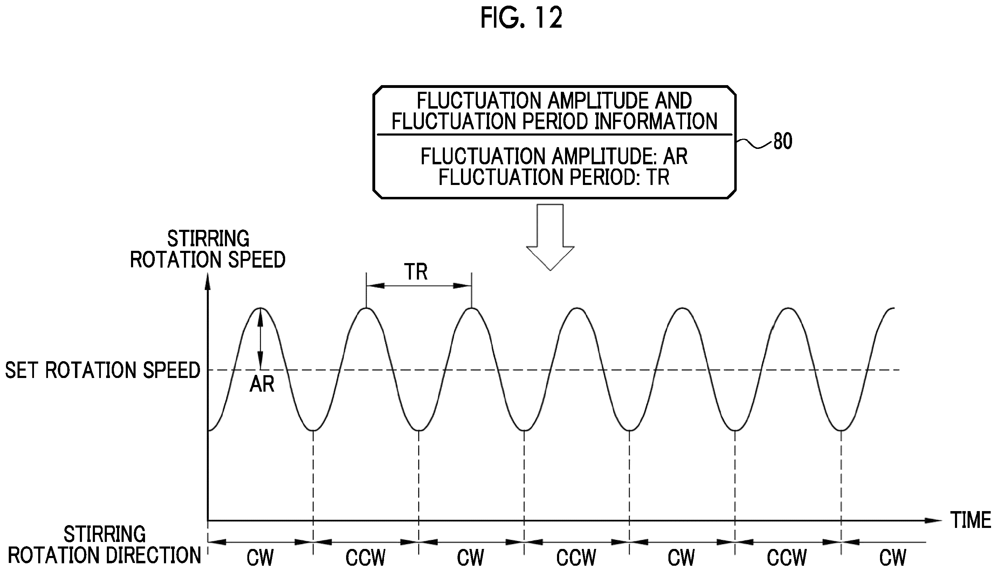

As shown in FIG. 12 as an example, not only the stirring rotation speed of the stirring bar 33 but also the stirring rotation direction of the stirring bar 33 may be periodically changed. FIG. 12 shows a case where the stirring rotation direction is switched to the clockwise CW and the counterclockwise CCW for each period. By periodically changing the stirring rotation direction of the stirring bar 33 in this way, the undissolved powdered culture medium PM is less likely to be deposited on the liquid surface of the liquid in the mixing container 16 and/or the bottom surface of the mixing container 16, and the concentration of the culture medium CM can be further stabilized.

The first embodiment and the third embodiment, the second embodiment and the third embodiment, or the first and second embodiments and the third embodiment may be implemented in combination.

In any of the first to third embodiments, existing culture medium preparation devices 10 such as the pure water supply pump 22, the stirrer 32, and the culture medium supply pump 36 are used in order to apply an appropriate stimulus to the liquid in the mixing container 16. Therefore, it is possible to suppress an increase in cost and a large scale of the culture medium preparation device 10 as compared with an aspect in which a new device for applying an appropriate stimulus to the liquid in the mixing container 16 is added.

### [Examples]

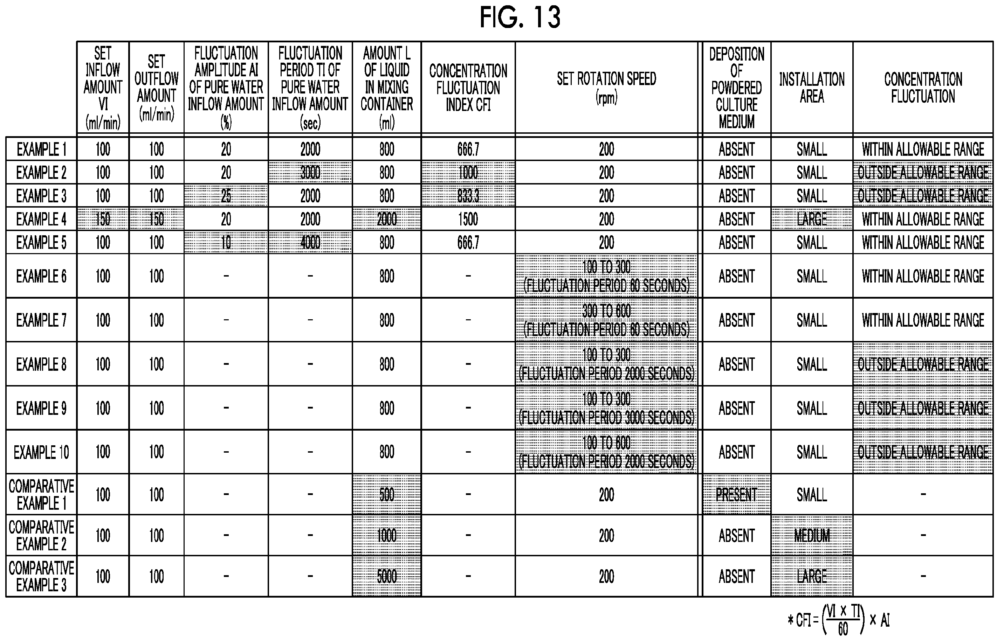

FIG. 13 shows a table 85 summarizing Examples and Comparative Examples. First, the devices used in Examples and Comparative Examples are listed. A beaker manufactured by HARIO CO., LTD. was used as the mixing container 16. As the powdered culture medium supply machine 17, a powder weighing and feeding machine (model number: PSF-100SA) manufactured by AS ONE Corporation was used. A stirrer used to crush rat holes in the hopper 30 was Tornado (stirrer) with a timer (model number: SMT-104) manufactured by AS ONE Corporation.

For each flow path such as the pure water supply line 21, the culture medium supply line 35, and the waste liquid line 42, a lab silicone tube (product number: 9-869-07, inner diameter × outer diameter = φ4 mm × φ6 mm) manufactured by AS ONE Corporation or the like was used after being appropriately cut.

A Masterflex pump (model number: 07528-30) manufactured by Yamato Scientific Co., Ltd. was used as a driving unit of the pure water supply pump 22 and the culture medium supply pump 36. A Masterflex pump head (model number: 77201-60) manufactured by Yamato Scientific Co., Ltd. was used as a pump head of the pure water supply pump 22 and the culture medium supply pump 36. In the driving units of the pure water supply pump 22 and the culture medium supply pump 36, the above-described Masterflex pump was shared for both purposes, and two of the above-described Masterflex pump heads were connected in tandem to one Masterflex pump.

A clamp-on type flow sensor (model number: FD-XA1) manufactured by KEYENCE CORPORATION was used as the pure water flow meter 23 and the culture medium flow meter 37.

An Ultra stirrer (model number: MSD-1) manufactured by AS ONE Corporation was used as the stirrer 32. As the stirring bar 33, a regular stirring bar (model number: C 8 × 30, diameter × length = φ8 mm × 30 mm) manufactured by AS ONE Corporation was used.

As the main bodies of the conductivity meter 39 and the hydrogen-ion exponent meter 40, a benchtop potential hydrogen (pH) and electrical conductivity meter (model number: F-74) manufactured by Horiba, Ltd. was also shared for both purposes. As the electrode of the conductivity meter 39, a general-purpose electrical conductivity cell (model number: 3562-10D) manufactured by HORIBA, Ltd. was used. As an electrode of the hydrogen-ion exponent meter 40, a non-refillable pH electrode (model number: 9652-20D) manufactured by HORIBA, Ltd. was used. In all of these electrodes, the conductivity and the hydrogen-ion exponent can be measured inline. As the flow cell installed together with these electrodes, a flow cell (product number: 3200844642) manufactured by HORIBA, Ltd. was used.

Subsequently, the materials used in Examples and Comparative Examples are listed. As the powdered culture medium PM, a minimum essential medium (MEM) (product number: 41500083) manufactured by Gibco was used.

Finally, experimental conditions of Examples and Comparative Examples are listed. The set inflow amount of the pure water PW and the set outflow amount of the culture medium CM are 100 ml/min unless otherwise specified. The supply amount of the powdered culture medium PM to the mixing container 16 per unit time is 0.76 g/min. The rotation speed of the screw of the feeder 31 of the powdered culture medium supply machine 17 is 1 rotation per minute (rpm). The set rotation speed of the stirring bar 33 by the stirrer 32 is 200 rpm unless otherwise specified. The measurement interval of the conductivity meter 39 and the hydrogen-ion exponent meter 40 is 10 seconds.

The flow rate of the pure water PW was calculated from the supply amount of the powdered culture medium PM, with a value that matched the "amount of water to be put in with respect to a certain amount of powder" described in the instruction manual of the powdered culture medium PM. As the supply amount of the powdered culture medium PM, a value obtained by actually supplying the powdered culture medium PM by rotating the screw of the feeder 31 at a set rotation speed and measuring the weight of the powdered culture medium PM supplied in a unit time with an electronic balance was used. As the electronic balance, a compact balance (model number: EW-150i) manufactured by A&D Company, Limited was used.

As described above, since the inflow amount of the pure water PW into the mixing container 16 and the outflow amount of the culture medium CM from the mixing container 16 are appropriately controlled, the amount of the liquid in the mixing container 16 does not change from the beginning to the end and is a value set in the setting experiment.

Examples 1 to 5 are examples in which the first embodiment is adopted and the inflow amount of the pure water PW into the mixing container 16 is periodically fluctuated. Example 1 is a case where the inflow amount of the pure water PW into the mixing container 16 was periodically fluctuated with a fluctuation amplitude AI of 20% and a fluctuation period TI of 2000 seconds. In this case, the amount of the liquid in the mixing container 16 was 800 ml. In addition, the concentration fluctuation index CFI in this case was 666.7, which was equal to or less than the amount of the liquid in the mixing container 16. Then, the deposition of the undissolved powdered culture medium PM was "none", the installation area of the mixing container 16 was "small", and the concentration fluctuation of the culture medium CM due to the periodic fluctuation of the inflow amount of the pure water PW into the mixing container 16 was "within an allowable range". Therefore, Example 1 was the best.

Example 2 was a case where the fluctuation period TI was set to be longer than 2000 seconds of Example 1, that is, 3000 seconds. The amount of the liquid in the mixing container 16 in this case was also 800 ml as in Example 1. However, the concentration fluctuation index CFI in this case was 1000, which was larger than the amount of the liquid in the mixing container 16. The deposition of the undissolved powdered culture medium PM was "none", the installation area of the mixing container 16 was "small", and the same as in Example 1. However, the concentration fluctuation of the culture medium CM due to the periodic fluctuation of the inflow amount of the pure water PW into the mixing container 16 was "outside an allowable range". The reason why the concentration fluctuation was "outside an allowable range" was that the fluctuation period was set to be long.

Example 3 was a case where the fluctuation amplitude AI was set to be higher than 20% of Example 1, that is, 25%. The amount of the liquid in the mixing container 16 in this case was also 800 ml as in Examples 1 and 2. However, the concentration fluctuation index CFI in this case was 833.3, which was larger than the amount of the liquid in the mixing container 16. The deposition of the undissolved powdered culture medium PM was "none", the installation area of the mixing container 16 was "small", and the same as in Examples 1 and 2. However, the concentration fluctuation of the culture medium CM due to the periodic fluctuation of the inflow amount of the pure water PW into the mixing container 16 was "outside an allowable range" as in Example 2. The reason why the concentration fluctuation was "outside an allowable range" was that the fluctuation amplitude was set to be high.

In Example 4, the set inflow amount of the pure water PW and the set outflow amount of the culture medium CM were set to 150 ml/min. The fluctuation amplitude AI and the fluctuation period TI were the same as in Example 1. The amount of the liquid in the mixing container 16 in this case was 2000 ml. In addition, the concentration fluctuation index CFI in this case was 1500, which was equal to or less than the amount of the liquid in the mixing container 16. The deposition of the undissolved powdered culture medium PM was "none", the concentration fluctuation of the culture medium CM due to the periodic fluctuation of the inflow amount of the pure water PW into the mixing container 16 was "within an allowable range", but the installation area of the mixing container 16 was "large".

Example 5 was a case where the fluctuation amplitude AI was set to be lower than 20% of Example 1, that is, 10%, and the fluctuation period TI was set to be longer than 4000 seconds of Example 1, that is, 2000 seconds. The amount of the liquid in the mixing container 16 in this case was also 800 ml as in Example 1. In addition, the concentration fluctuation index CFI in this case was also 666.7 as in Example 1, which was equal to or less than the amount of the liquid in the mixing container 16. Then, the deposition of the undissolved powdered culture medium PM was "none", the installation area of the mixing container 16 was "small", and the concentration fluctuation of the culture medium CM due to the periodic fluctuation of the inflow amount of the pure water PW into the mixing container 16 was "within an allowable range". Therefore, Example 5 was also the best as in Example 1.

Examples 6 to 10 were examples in which the third embodiment was adopted and the stirring rotation speed of the stirring bar 33 by the stirrer 32 was periodically fluctuated. Example 6 was a case where the stirring rotation speed of the stirring bar 33 by the stirrer 32 was fluctuated at a fluctuation period TR = 60 seconds between 100 rpm and 300 rpm. The amount of the liquid in the mixing container 16 in this case was also 800 ml as in Example 1 and the like. Then, the deposition of the undissolved powdered culture medium PM was "none", the installation area of the mixing container 16 was "small", and the concentration fluctuation of the culture medium CM due to the periodic fluctuation of the stirring rotation speed of the stirring bar 33 by the stirrer 32 was "within an allowable range". Therefore, Example 6 was also the best as in Example 1 and the like.

Example 7 was a case where the stirring rotation speed of the stirring bar 33 by the stirrer 32 was fluctuated at a fluctuation period TR = 60 seconds between 300 rpm and 600 rpm. The amount of the liquid in the mixing container 16 in this case was also 800 ml as in Example 1 and the like. Then, the deposition of the undissolved powdered culture medium PM was "none", the installation area of the mixing container 16 was "small", and the concentration fluctuation of the culture medium CM due to the periodic fluctuation of the stirring rotation speed of the stirring bar 33 by the stirrer 32 was "within an allowable range". Therefore, Example 7 was also the best as in Example 1 and the like.

Example 8 was a case where the stirring rotation speed of the stirring bar 33 by the stirrer 32 was fluctuated at a fluctuation period TR = 2000 seconds between 100 rpm and 300 rpm as in Example 6. The amount of the liquid in the mixing container 16 in this case was also 800 ml as in Example 1 and the like. Then, the deposition of the undissolved powdered culture medium PM was "none", the installation area of the mixing container 16 was "small", but the concentration fluctuation of the culture medium CM due to the periodic fluctuation of the stirring rotation speed of the stirring bar 33 by the stirrer 32 was "outside an allowable range". The reason why the concentration fluctuation was "outside an allowable range" was that the fluctuation period was set to be long.

Example 9 was a case where the stirring rotation speed of the stirring bar 33 by the stirrer 32 was fluctuated at a fluctuation period TR = 3000 seconds between 100 rpm and 300 rpm as in Example 6. The amount of the liquid in the mixing container 16 in this case was also 800 ml as in Example 1 and the like. Then, the deposition of the undissolved powdered culture medium PM was "none", the installation area of the mixing container 16 was "small", but the concentration fluctuation of the culture medium CM due to the periodic fluctuation of the stirring rotation speed of the stirring bar 33 by the stirrer 32 was "outside an allowable range". The reason why the concentration fluctuation was "outside an allowable range" was that the fluctuation period was set to be long.

Example 10 was a case where the stirring rotation speed of the stirring bar 33 by the stirrer 32 was fluctuated at a fluctuation period TR = 2000 seconds between 100 rpm and 600 rpm. The amount of the liquid in the mixing container 16 in this case was also 800 ml as in Example 1 and the like. Then, the deposition of the undissolved powdered culture medium PM was "none", the installation area of the mixing container 16 was "small", but the concentration fluctuation of the culture medium CM due to the periodic fluctuation of the stirring rotation speed of the stirring bar 33 by the stirrer 32 was "outside an allowable range". The reason why the concentration fluctuation was "outside an allowable range" was that the fluctuation amplitude was set to be high and the fluctuation period was set to be long.

Examples in which the second embodiment was adopted, that is, examples in which the outflow amount of the culture medium CM from the mixing container 16 was periodically fluctuated were substantially the same as Examples 1 to 5 in which the first embodiment was adopted, and thus the description thereof will be omitted.

Comparative Examples 1 to 3 were examples in which the inflow amount of the pure water PW to the mixing container 16 was not periodically fluctuated, the outflow amount of the culture medium CM from the mixing container 16 was not periodically fluctuated, and the stirring rotation speed of the stirring bar 33 by the stirrer 32 was not periodically fluctuated, and only the amount of the liquid in the mixing container 16 was changed. Comparative Example 1 was a case where the amount of the liquid in the mixing container 16 was 500 ml. In this case, the deposition of the undissolved powdered culture medium PM was "present", and the installation area of the mixing container 16 was "small". The reason why the deposition of the undissolved powdered culture medium PM was "present" was that the amount of the liquid in the mixing container 16 was reduced.

Comparative Example 2 was a case where the amount of the liquid in the mixing container 16 was 1000 ml. In this case, the deposition of the undissolved powdered culture medium PM was "absent", but the installation area of the mixing container 16 was "medium". Comparative Example 3 was a case where the amount of the liquid in the mixing container 16 was 5000 ml. In this case, the deposition of the undissolved powdered culture medium PM was "absent", but the installation area of the mixing container 16 was "large".

In Comparative Examples 2 and 3, the result of "absent" was obtained for the deposition of the undissolved powdered culture medium PM. However, the amount of the liquid in the mixing container 16 of Comparative Example 2 was 1000 ml, the amount of the liquid in the mixing container 16 of Comparative Example 3 was 5000 ml, and both were larger than 800 ml of Examples 1 to 3 and 5 to 10. Therefore, Comparative Examples 2 and 3 were inferior to Examples 1 to 3 and 5 to 10 in terms of the requirement for the installation area. In other words, by periodically changing the inflow amount of the pure water PW into the mixing container 16, periodically changing the outflow amount of the culture medium CM from the mixing container 16, or periodically changing the stirring rotation speed of the stirring bar 33 by the stirrer 32, the amount of the liquid in the mixing container 16 can be reduced, and the reduction in the installation area can be contributed. In a case where there is no restriction on the installation area, the configuration of Comparative Example 2 or 3 may be adopted.

From the above, it was confirmed that in Examples 1 to 10 according to the technology of the present disclosure, the undissolved powdered culture medium PM was less likely to be deposited, and the stabilization of the concentration of the culture medium CM was contributed. In addition, it was found that Examples 1 to 3 and 5 to 10 according to the technology of the present disclosure contributed to the reduction in the installation area of the mixing container 16. Further, according to Example 1 and Examples 4 to 7, it was found that the concentration fluctuation of the culture medium CM was within the allowable range. In particular, according to Example 1, Example 4, and Example 5 in which the concentration fluctuation index CFI was set to be equal to or less than the amount L of the liquid in the mixing container 16, it was found that the concentration fluctuation of the culture medium CM due to the periodic fluctuation of the inflow amount of the pure water PW into the mixing container 16 was within the allowable range.

There are many types of powdered culture media PM. The appropriate values of the fluctuation amplitude, the fluctuation period, and the amount of the liquid in the mixing container 16 differ depending on the type of the powdered culture medium PM. Therefore, the operator performs the setting experiment each time the type of the powdered culture medium PM is changed to set the appropriate values of the fluctuation amplitude, the fluctuation period, and the amount of the liquid in the mixing container 16.

The characteristics of the culture medium CM to be supplied to the culture tank 11, which are to be measured inline, are not limited to the exemplified conductivity and hydrogen-ion exponent. In addition to or instead of these, a Raman spectrum, an infrared absorption spectrum, a near infrared absorption spectrum, an ultraviolet absorption spectrum, a fluorescence spectrum, and the like may be measured.

The method of measuring the timing at which the discard of the culture medium CM is stopped and the supply of the culture medium CM to the culture tank 11 is started is not limited to the method based on the conductivity in each of the embodiments. The time taken for the concentration of the culture medium CM to reach the set concentration is measured and stored in advance. Then, in a case where the elapsed time from the start of the continuous supply of the pure water PW and the powdered culture medium PM to the mixing container 16 is timed and the elapsed time reaches the stored time, the discard of the culture medium CM may be stopped and the supply of the culture medium CM to the culture tank 11 may be started. In this case, the conductivity meter 39 is not necessary.

In each of the embodiments, pure water PW is exemplified as the solvent and the purified water, but the present disclosure is not limited thereto. Distilled water or the like may be used. In addition, in each of the above-described embodiments, the culture medium CM is described as an example of the solution, but the present disclosure is not limited thereto. The solution may be a buffer solution. Therefore, the powder is not limited to the powdered culture medium PM described as an example, and may be a powder constituting the solid component of the buffer solution.

In each of the above-described embodiments, for example, as the hardware structure of the processing unit that executes various types of processing, such as the pure water supply pump drive control unit 65, the powdered culture medium supply machine drive control unit 66, the stirrer drive control unit 67, the culture medium supply pump drive control unit 68, and the three-way valve drive control unit 69, various processors described later can be used. As described above, the various processors include, in addition to the CPU 51 that is a general-purpose processor executing software (operation program 60) to function as various processing units, a programmable logic device (PLD) that is a processor of which a circuit configuration can be changed after manufacture, such as a field programmable gate array (FPGA), and a dedicated electrical circuit that is a processor having a circuit configuration which is designed for exclusive use to execute specific processing, such as an application specific integrated circuit (ASIC).

One processing unit may be configured by one of the various processors, or may be configured by a combination of two or more processors of the same type or different types (for example, a combination of a plurality of FPGAs and/or a combination of a CPU and an FPGA). In addition, a plurality of processing units may be configured by one processor.

As an example in which the plurality of processing units are configured by one processor, first, as represented by a computer such as a client and a server, there is a form in which one processor is configured by a combination of one or more CPUs and software and the processor functions as the plurality of processing units. Second, as represented by a system on chip (SoC) or the like, there is a form in which a processor that realizes the functions of the entire system including a plurality of processing units by one integrated circuit (IC) chip is used. In this manner, various processing units are configured by using one or more of the various processors as a hardware structure.

Further, as a hardware structure of the various processors, more specifically, an electrical circuit (circuitry) in which circuit elements such as semiconductor elements are combined can be used.

The technology according to the following supplementary notes can be understood from the above description.

### [Supplementary Note 1]

A solution preparation device including a first pump that causes a solvent to continuously flow into a mixing container, a powder feeder that continuously supplies a powder to the mixing container, a stirrer that stirs the solvent and the powder in the mixing container, and a second pump that causes a solution having a set concentration, which is prepared by mixing the solvent and the powder, to continuously flow out from the mixing container, the solution preparation device comprising:
a processor that controls driving of the first pump, the powder feeder, the stirrer, and the second pump,
in which the processor is configured to periodically change at least any one of an inflow amount of the solvent into the mixing container by the first pump, an outflow amount of the solution from the mixing container by the second pump, or a stirring rotation speed by the stirrer.

### [Supplementary Note 2]

The solution preparation device according to Supplementary Note 1,
in which a fluctuation amplitude and a fluctuation period of at least any one of the inflow amount, the outflow amount, or the stirring rotation speed are set to values at which a concentration fluctuation of the solution caused by the periodic fluctuation of at least any one of the inflow amount, the outflow amount, or the stirring rotation speed is within an allowable range.

### [Supplementary Note 3]

The solution preparation device according to Supplementary Note 2,
in which a concentration fluctuation index CFI represented by the following equation is equal to or less than an amount L of a liquid in the mixing container, Equation: $CFI = \left\{\left(V\times T\right)/60\right\} \times A ,$ here, V is a set amount of the inflow amount or the outflow amount, A is a fluctuation amplitude of at least any one of the inflow amount or the outflow amount, and T is a fluctuation period of at least any one of the inflow amount or the outflow amount.

### [Supplementary Note 4]

The solution preparation device according to any one of Supplementary Notes 1 to 3,
in which the processor is configured to further periodically change a stirring rotation direction by the stirrer in a case where the processor is configured to periodically change the stirring rotation speed.

### [Supplementary Note 5]

The solution preparation device according to any one of Supplementary Notes 1 to 4, in which the solvent is purified water, the powder is a powdered culture medium, and the solution is a culture medium.

The technology of the present disclosure can also appropriately combine various embodiments and/or various modification examples described above. It goes without saying that the present disclosure is not limited to each of the embodiments described above, and various configurations can be employed without departing from the gist. Furthermore, the technology of the present disclosure extends to a storage medium that non-transitorily stores the program, and a computer program product including the program, in addition to the program.

The description content and the shown content described above are detailed descriptions of parts related to the technology of the present disclosure and are merely an example of the technology of the present disclosure. For example, the above description of the configuration, the function, the action, and the effect are the description of examples of the configuration, the function, the action, and the effect of the parts according to the technology of the present disclosure. Accordingly, it goes without saying that unnecessary parts may be deleted, new elements may be added, or replacements may be made with respect to the description content and the shown content described above within a range that does not depart from the gist of the technology of the present disclosure. Further, in order to avoid complication and facilitate understanding of the parts according to the technology of the present disclosure, the description related to common general knowledge not requiring special description in order to implement the technology of the present disclosure is omitted in the description content and the shown content described above.

In the present specification, "A and/or B" is synonymous with "at least one of A or B". That is, "A and/or B" means that it may be only A, only B, or a combination of A and B. In addition, in the present specification, in a case where three or more matters are expressed by being connected by "and/or", the same concept as "A and/or B" is applied.

All documents, patent applications, and technical standards described in the present specification are incorporated in the present specification by reference to the same extent as in a case where each of the documents, the patent applications, and the technical standards are specifically and individually indicated to be incorporated by reference.

## Claims

1. A solution preparation device including a first pump that causes a solvent to continuously flow into a mixing container, a powder feeder that continuously supplies a powder to the mixing container, a stirrer that stirs the solvent and the powder in the mixing container, and a second pump that causes a solution having a set concentration, which is prepared by mixing the solvent and the powder, to continuously flow out from the mixing container, the solution preparation device comprising:
a processor that controls driving of the first pump, the powder feeder, the stirrer, and the second pump, the processor being configured to:
periodically change at least any one of an inflow amount of the solvent into the mixing container by the first pump, an outflow amount of the solution from the mixing container by the second pump, or a stirring rotation speed by the stirrer.

2. The solution preparation device according to claim 1, wherein a fluctuation amplitude and a fluctuation period of at least any one of the inflow amount, the outflow amount, or the stirring rotation speed are set to values at which a concentration fluctuation of the solution caused by the periodic fluctuation of at least any one of the inflow amount, the outflow amount, or the stirring rotation speed is within an allowable range.

3. The solution preparation device according to claim 2, wherein a concentration fluctuation index CFI represented by the following equation is equal to or less than an amount L of a liquid in the mixing container, Equation: $CFI = \left\{\left(V\times T\right)/60\right\} \times A ,$ here, V is a set amount of the inflow amount or the outflow amount, A is a fluctuation amplitude of at least any one of the inflow amount or the outflow amount, and T is a fluctuation period of at least any one of the inflow amount or the outflow amount.

4. The solution preparation device according to claim 1, wherein the processor is configured to further periodically change a stirring rotation direction by the stirrer in a case where the processor is configured to periodically change the stirring rotation speed.

5. The solution preparation device according to claim 1, wherein:
the solvent is purified water,
the powder is a powdered culture medium, and
the solution is a culture medium.

6. An operation method of a solution preparation device including a first pump that causes a solvent to continuously flow into a mixing container, a powder feeder that continuously supplies a powder to the mixing container, a stirrer that stirs the solvent and the powder in the mixing container, and a second pump that causes a solution having a set concentration, which is prepared by mixing the solvent and the powder, to continuously flow out from the mixing container, the method comprising:
controlling driving of the first pump, the powder feeder, the stirrer, and the second pump; and
periodically changing at least any one of an inflow amount of the solvent into the mixing container by the first pump, an outflow amount of the solution from the mixing container by the second pump, or a stirring rotation speed by the stirrer.

7. An operation program of a solution preparation device including a first pump that causes a solvent to continuously flow into a mixing container, a powder feeder that continuously supplies a powder to the mixing container, a stirrer that stirs the solvent and the powder in the mixing container, and a second pump that causes a solution having a set concentration, which is prepared by mixing the solvent and the powder, to continuously flow out from the mixing container, the program causing a computer to execute a process comprising:
controlling driving of the first pump, the powder feeder, the stirrer, and the second pump; and
periodically changing at least any one of an inflow amount of the solvent into the mixing container by the first pump, an outflow amount of the solution from the mixing container by the second pump, or a stirring rotation speed by the stirrer.
